(19) Europäisches Patentamt
European Patent Office
Office européen des brevets

(11) **EP 1 879 021 A2**

(12) **EUROPEAN PATENT APPLICATION**

(43) Date of publication:
**16.01.2008 Bulletin 2008/03**

(51) Int Cl.:
***G01N 23/04*** *(2006.01)*

(21) Application number: **07252704.7**

(22) Date of filing: **05.07.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **14.07.2006 JP 2006194073**

(71) Applicant: **Konica Minolta Medical & Graphic, Inc.**
**Tokyo 163-0512 (JP)**

(72) Inventors:
• **Shinden, Yuko,**
**c/o Konica Minolta Medical &Graphic.Inc.**
**Tokyo, 192-8505 (JP)**
• **Ohara, Hiromu,**
**c/o Konica Minolta Medical & Graphic, Inc.**
**Tokyo, 192-8505 (JP)**

(74) Representative: **Alton, Andrew et al**
**Urquhart-Dykes & Lord LLP**
**Tower North Central**
**Merrion Way**
**Leeds LS2 8PA (GB)**

(54) **Phase contrast radiography with a microfocus tube providing X-rays having an average energy between 10 and 20 keV**

(57) An X-ray source, a test substance holding section and an image recording section are arranged and structured to conduct a phase contrast radiographing such that when R1 represents a distance (m) from a focal point of the X-ray source to the test substance held by the test substance holding section and R3 represents a distance (m) from the focal point of the X-ray source to the X-ray detector, a magnifying power M represented by the formula of (M = R3/R1) is from 10 to 40 and an X-ray arrival ratio of an amount of X-rays having arrived to the X-ray detector to an amount of the emitted X-rays is 45 % or more.

FIG. 1( a )

EP 1 879 021 A2

**Description**

**CROSS-REFERENCE TO RELATED APPLICATION**

This application is based on Japanese Patent Application No. 2006-194073 filed on July 14, 2006, in Japanese Patent Office, the entire content of which is hereby incorporated by reference.

**BACKGROUND OF THE INVENTION**

The present invention relates to an X-ray radiographing apparatus for capturing a radiograph.

In recent years, asbestos infiltrated into the human body has become an issue on the point that critical diseases such as pneumoconiosis and mesothelioma are induced by the asbestos. Therefore, there has been introduced a checkup wherein a patient is radiographed to obtain a radiograph, and presence of asbestos in the human body is detected from this radiograph. Owing to the detection of the asbestos, it is expected that sideration can be controlled through rapid treatment (medical treatment and preventive measure).

However, it is difficult to make a microscopic object having a diameter of about 10 $\mu$m or less such as asbestos to be a visible image in commonly used X-ray radiographing apparatus that is used to radiograph a chest and the limbs. Even if a visible image is acquired, visibility is low, a medial doctor cannot observe (distinguish) in many cases. The reason for this is that a size of the object to be radiogrphed is smaller than spatial resolution of an image recording apparatus used in the X-ray radiographing apparatus.

For acquiring a radiograph on which a microscopic object such as asbestos can be observed finely, enlarging radiographing is effective. The enlarging radiographing is a radiographing method to acquire a radiograph that is enlarged to be greater than an actual photographic object, by causing a focus size of X-ray tube, a distance from the X-ray tube to a photographic object and a distance from a photographic object to the image recording apparatus to be in the prescribed relationship.

For the further improvement of visibility in the enlarging radiographing, it is considered to increase an amount of X-ray to be irradiated by enhancing magnifying power in the course of enlarging radiographing, or by causing the focus size of X-ray tube to be greater. Even in the past, there has been disclosed a method to obtain digital image data by conducting enlarging radiographing at magnifying power of 10x and by reading the radiograph thus obtained at the reading sampling pitch of 25 $\mu$m, because an object to be radiographed is a small animal (for example, see Japanese Patent Publication Open to Public Inspection No. 10-268450).

There is further disclosed a method to enhance the magnifying power by causing a distance from a photographic object to an image recording apparatus to be 0.3 m or more (for example, see International Patent Publication Open to Public Inspection No. 11-502620).

When radiographing the human body, especially, asbestos in the chest, it is necessary to adjust radiographing conditions by taking contrast between the asbestos and the human body tissue into consideration. However, when the asbestos in the test substance picked from the human body is made to be an object to be radiographed, an influence of the human body tissue to the asbestos is small. Therefore, even when radiographing conditions and image processing conditions for the human body representing a photographic object are employed, the visibility of the asbestos is not always improved in the radiograph. Further, when the test substances (specimen materials) is made to be a photographic object, it is not necessary to consider a problem of radiation exposure, which is different from an occasion where the human body is made to be a photographic object. Even if the radiographing time is long, motion artifact (blurring) caused by movement of a photographic object in the course of radiographing is not caused. It is therefore possible to adjust radiographing conditions so that the visibility of asbestos in the radiograph may become the best.

**SUMMARY**

An objective of the invention is to provide an X-ray radiographing apparatus that is capable of acquiring a radiograph wherein visibility of an asbestos is high, when the asbestos contained in the test substance is made to be an object to be radiographed.

The above object can be attained by the structure of the X-ray radiographing apparatus described in the following items.

Item 1. An X-ray radiographing apparatus, comprising:

(1) a radiographing section having an X-ray source provided with a focus size D ($\mu$m) of from 1 to 30 and for emitting X-rays having an X-ray energy (keV) of from 10 to 20;
(2) a test substance holding section for holding a test substance;

(3) an image recording section for accommodating a X-ray detector;

wherein the X-ray source, the test substance holding section and the image recording section are arranged and structured to conduct a phase contrast radiographing such that when R1 represents a distance (m) from a focal point of the X-ray source to the test substance held by the test substance holding section and R3 represents a distance (m) from the focal point of the X-ray source to the X-ray detector, a magnifying power M represented by the formula of (M = R3/R1) is from 10 to 40 and an X-ray arrival ratio of an amount of X-rays having arrived to the X-ray detector to an amount of the emitted X-rays is 45 % or more.

Item 2. The X-ray radiographing apparatus of Item 1, wherein the X-ray arrival ratio is 50 % or more.

Item 3. The X-ray radiographing apparatus of Item 1, wherein the X-ray energy (keV) is from 10 to 15.

Item 4. The X-ray radiographing apparatus of Item 1, wherein the image recording section is arranged to satisfy the following formula.

$$0.5 < R3 < 4 \ (m)$$

Item 5. The X-ray radiographing apparatus of Item 4, wherein the image recording section is arranged to satisfy the following formula.

$$2 < R3 < 3 \ (m)$$

Item 6. The X-ray radiographing apparatus of Item 1, wherein the magnifying power M is from 15 to 35.

Item 7. The X-ray radiographing apparatus of Item 6, wherein the magnifying power M is from 18 to 28.

Item 8. The X-ray radiographing apparatus of Item 1, wherein the test substance holding section and the image recording section are located below the radiographing section so as to make the distances R1 and R3 adjustable respectively.

Item 9. The X-ray radiographing apparatus of Item 1, wherein the image recording section includes a casing, the casing has a front plate to cover a irradiation surface of the X-ray detector, and the front plate is structured to be removable at the time of the phase contrast radiographing. Item 10. The X-ray radiographing apparatus of Item 1, wherein the test substance holding section has an opening not to shield an X-ray irradiation region of the test substance. Item 11. The X-ray radiographing apparatus of Item 1, wherein the X-ray detector is a phosphor plate.

Item 12. The X-ray radiographing apparatus of Item 1, wherein the X-ray detector is a semiconductor element plate.

Item 13. The X-ray radiographing apparatus of Item 1, wherein the size ($\mu$m) of the test substance is 0.05 to 50.

## BRIEF DESCRIPTION OF THE DRAWINGS

Fig. 1(a) is a diagram showing the structure of the X-ray radiographing apparatus in the present embodiment, and Fig. 1(b) is a plan view showing a table 8 provided with an opening 9.

Fig. 2(a) is a perspective view showing the structure of the cassette. Fig. 2(b) is a cross-sectional view of the cassette shown in Fig. 2(a).

Fig. 3 is a diagram illustrating phase contrast radiographing and a phase contrast effect.

Fig. 4 is a diagram showing the relationship between edge strength in phase contrast radiography and unsharpness.

Fig. 5 is a diagram illustrating the occasion wherein unsharpness is caused in phase contrast radiography.

Fig. 6 is a diagram of characteristics showing relationship between X-ray energy and refraction difference $\delta$.

Fig. 7 is a diagram of characteristics showing relationship between X-ray energy and X-ray arrival ratio (X-ray arrival ratio to phosphor plate, X-ray arrival ratio for front plate).

Fig. 8 is a diagram of characteristics showing the relationship between a distance for radiographing and an X-ray-ray transport factor.

## DETAILED DESCRIPTION OF THE PREFERRED EMBODIMENT

In the present example, there will be given an explanation wherein phase contrast radiographing is conducted by irradiating X-ray having low energy to the test substance, and an image recording section and/or a radiographing distance from an X-ray source to an X-ray detector is structured so that an X-ray arrival ratio of an amount of X-rays having arrived to the X-ray detector of the image recording section to an amount of X-rays emitted from the X-ray source may become 45% or more.

<Structure of X-ray radiographing apparatus>

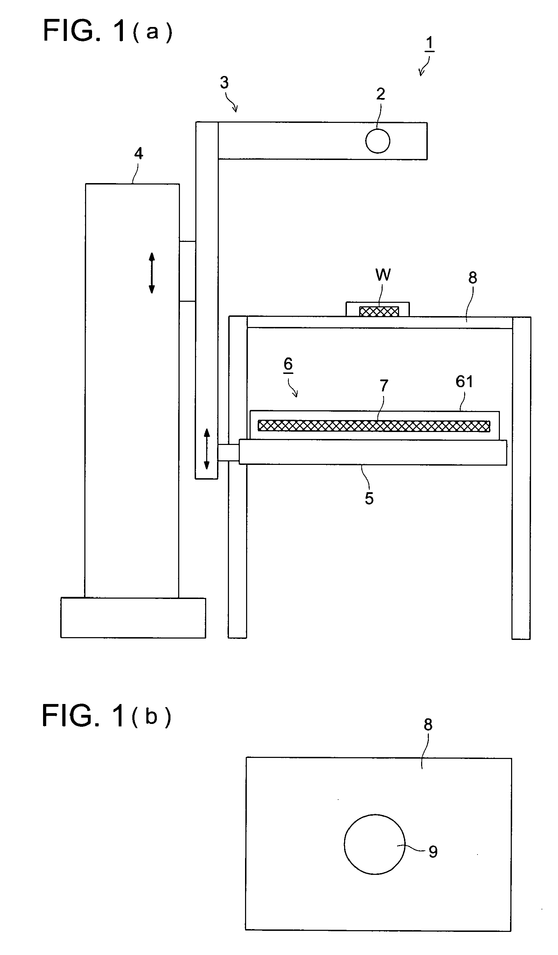

Fig. 1(a) shows the structure of X-ray radiographing apparatus 1 in the present embodiment.

The X-ray radiographing apparatus 1 is one to conduct phase contrast radiographing for an object to be radiographed of asbestos contained in test substance W such as tissue specimen picked from a patient. The X-ray radiographing apparatus 1 is constructed to be radiographing conditions under which a radiograph having high visibility of asbestos shading.

As shown in Fig. 1(a), the X-ray radiographing apparatus 1 is composed of radiographing section 3 for conducting radiographing and of main body portion 4 for controlling radiographing.

The radiographing section 3 is formed to be in an arm shape, and is constructed to be capable of rising and falling with the main body portion 4 serving as a supporting column. On the arm portion of the radiographing section 3, there are arranged X-ray source 2 and holding section 5 to face each other. The holding section 5 holds image recording section 6 and fixes its position. The holding section 5 is constructed to be capable of rising and falling along the supporting column of the radiographing section 3. It is possible to adjust a radiographing distance (each distance for X-ray source 2, test substance W and phosphor plate 7 in image recording section 6) by causing the radiographing section 3 and the holding section 5 to rise or fall and thereby by changing their heights.

The main body portion 4 controls a height of the radiographing section 3 and a height of the holding section 5, for phase contrast radiographing, so that a distance from X-ray source 2 to phosphor plate 2 (which is R3 (m)) may be within a range of 0.5 < R3 < 4. This distance R3 is preferably in a range of 2 < R3 < 3.

The X-ray source 2 is constructed to be composed of X-ray tube that irradiate X-ray whose X-ray energy is within a range from 10keV to 20keV. Under the assumption that a focus size (the size of focal point) of this X-ray tube to emit X-ray is represented by D ($\mu$m), it is preferable that a tube satisfying $1 \leq D \leq 30$ is used for the X-ray tube.

The test substance W to be radiographed is a tissue specimen picked from the human body, and it is put in a laboratory dish to be used for radiographing. The test substance W is set between X-ray source 2 and image recording section 6 by a test substance holding section such as a table 8. Incidentally, it is preferable that the table is composed of carbon having small X-ray absorption factor, and its thickness is reduced, for improving X-ray arrival ratio for image recording section 6. It is further possible to support test substance W by tongs in place of the table 8. Further, as shown in Fig. 1(b), an opening 9 is provided on the table 8. In this structure, the test substance W is placed at a position corresponding to the opening 9 by holding a periphery of the test substance W so as not to shield an irradiation region of the test substance W, thereby avoiding the X-ray absorption by the test substance holding section.

On the other hand, the main body portion 4 is constructed to be composed of a computer having therein CPU (Central Processing Unit), RAM (Random Access Memory), ROM (Read Only Memory) and an operation section. The main body portion 4 controls the radiographing section 3 and the holding section 5 to rise and fall, following the operation instruction, and controls irradiation operations of X-ray by X-ray source.

The image recording section 6 is one wherein phosphor plate 7 is housed in a casing that is called cassette 61. The phosphor plate 7 is an X-ray detector which absorbs and stores up X-ray energy, and image recording section 6 detects X-ray irradiated from X-ray source 2 and reaches through test substance W with phosphor plate 7. After that, phosphor plate 7 loaded in a reading apparatus while the phosphor plate 7 is housed in cassette 61, and image visualizing is conducted. The reading apparatus is one for irradiating excitation light such as a laser beam on the phosphor plate 7, then, for converting stimulating light emitted from phosphor plate 7 into image signal photoelectrically and for generating its image signal.

Cassette 61 having the structure wherein a front plate on the X-ray irradiating surface side of phosphor plate 7 can be removed is preferable. The reason for this is that the phosphor plate 7 can be replaced simply, and the front plate on the X-ray irradiating surface side which may obstruct X-ray transmission in the course of radiographing can be removed easily.

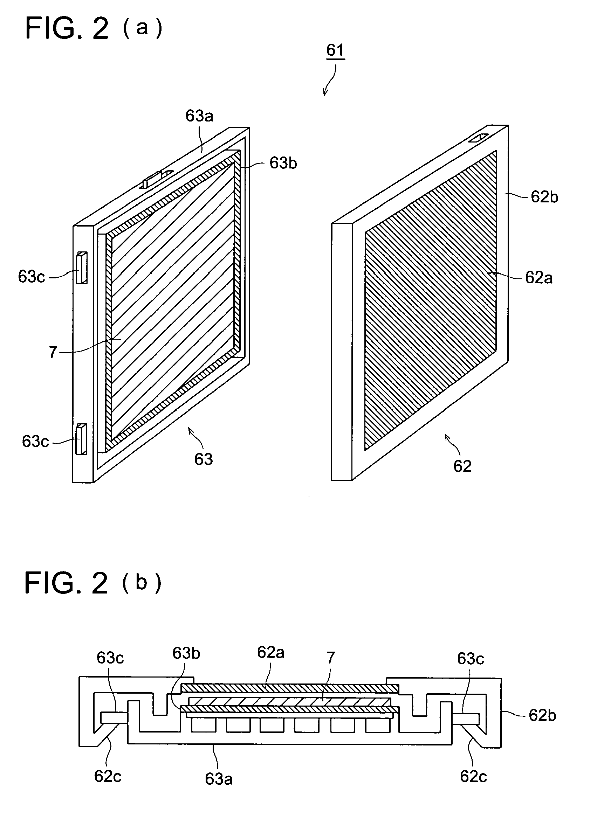

An example of the foregoing is shown in Fig. 2 (a). Cassette 61 shown in Fig. 2 (a) is composed of front casing 62 and rear casing 63 which can be separated, and the phosphor plate 7 is interposed between the front casing 62 and the rear casing 63 to be housed therein.

A circumference of the phosphor plate 7 is covered by a protective film composed of PET (polyethylene terephthalate).

Front casing 62 representing a casing portion on the X-ray irradiation surface side is formed by front plate 62a that covers X-ray irradiation surface of phosphor plate 7 and by frame 62b made of aluminum or hardened plastic which surrounds outer circumference of the front plate. The front plate 62a has a role of shielding against entrance of outer light into phosphor plate 7 and a role of protection against external shock. As a material of the front plate 62a, carbon whose X-ray absorption factor is relatively low and aluminum are used.

The rear casing 63 is composed of casing 63a that houses therein phosphor plate 7 and of supporting plate 63b that fixes the position of that housing.

Fig. 2(b) shows a sectional view of cassette 61 shown in Fig. 2(a).

On each of the front casing 62 and the rear casing 63, there is provided a lock mechanism. As shown in Fig. 2 (b), an arrangement is made so that engagement claw 63c provided to be outside of a side face of the rear casing 63 is protruded by the locking operation to the outside of the casing 63a, and is retreated by the lock releasing operation to the inside of the casing 63a. On the other hand, a tip of the frame 62b of the front casing 62 is formed to be in a form of a hook in terms of a cross section. Namely, the engagement claw 63c protruded by the locking operation into the inside of the side face of the front plate 62a engages with a hook portion of the front plate 62a.

Incidentally, the structure disclosed in Japanese Patent Publication Open to Public Inspection No. 2002-156717 can be used for the aforesaid cassette structure. Further, the structure capable of housing therein a column crystal plate disclosed in Japanese Patent Publication Open to Public Inspection No. 2005-106783 can also be employed.

For phase contrast radiographing, an article from which the front casing 62 has been removed is used, because radiographing for cassette 61 is conducted by removing the front plate 62a. Or, when radiographing by attaching the front plate 62a, those wherein a material and a thickness of the front plate 62a are adjusted so that X-ray arrival ratio from X-ray source 2 to phosphor plate 7 may be 45% or more, are used.

In the meantime, though an example of image recording section 6 wherein phosphor plate 7 is housed in cassette 61 is explained in the present embodiment, it is also possible to apply FPD (Flat Panel Detector) as the image recording section 6.

The FPD is a plate (semiconductor element plate) wherein sensing elements generating electric signals in accordance with an amount of X-ray entered are arranged in a form of a matrix, and it is different from the aforesaid phosphor plate 7 on the point that electric signals are generated directly in FPD. When the FPD is applied, electric signals generated in the FPD are subjected to A/D conversion, and digital image data thus obtained are outputted to main body section 4. In addition, FPD is also housed in a casing to be used for radiographing. In the same way as in cassette 61, the casing wherein the front section of a casing portion on an X-ray irradiation surface can be removed is preferable.

<Phase contrast radiographing and radiographing conditions>

Next, phase contrast radiographing by the aforesaid X-ray radiographing apparatus 1 will be explained.

The phase contrast radiographing is one wherein radiographing conditions such as a radiographing arrangement and irradiation conditions of X-ray are adjusted so that edge enhancement effect may be obtained, which is different from ordinary macrophotography.

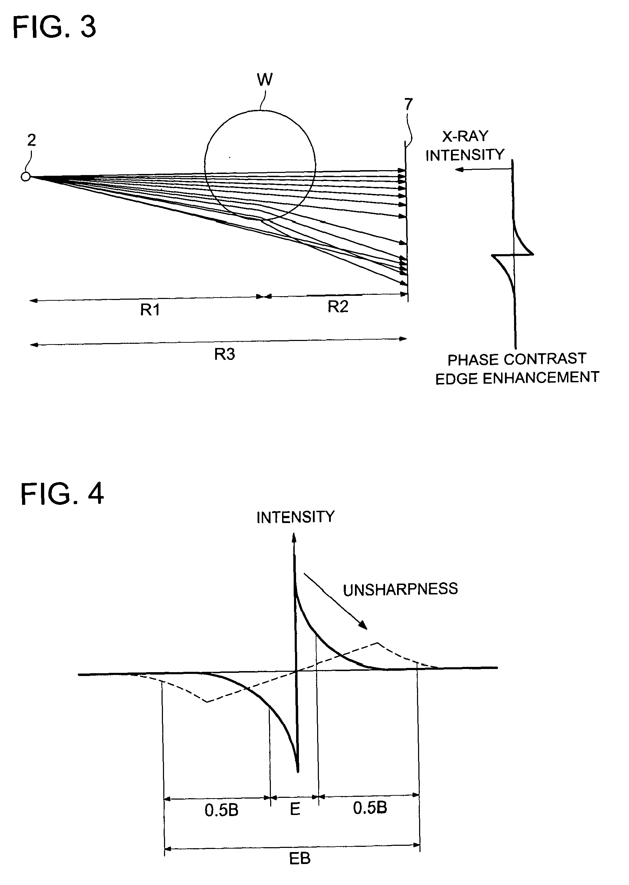

Fig. 3 is a diagram for illustrating an outline of phase contrast radiographing.

In the case of ordinary radiographing method, image recording section 6 is arranged at the position that is adjacent to a photographic object, and it is constituted to detect X-ray immediately after being transmitted through a photographic object with phosphor plate 7. Therefore, its radiograph is in a size that is substantially the same as a life size (a size which is the same as that of the photographic object).

In contrast to this, in the phase contrast radiographing, image recording section 6 is arranged so that a distance may be provided between test substance W representing a photographic object and phosphor plate 7 as shown in Fig. 3. Owing to this, a radiograph that is enlarged to be larger than a life size is obtained by X-ray emitted from X-ray source 2 in a shape of a cone beam.

In this case, magnifying power M of the enlarged radiograph to the life size can be obtained by the following expression (1). In the expression (1), R1 represents distance (m) from X-ray source 2 to test substance W, R2 represents distance (m) from test substance W to image recording section 6, test substance W, and R3 (R3 = R1 + R2) represents distance (m) from X-ray source 2 to phosphor plate 7 in image recording section 6.

$$M = R3/R1 \qquad (1)$$

In the radiograph created by phase contrast radiographing, X-ray that is refracted when it passes through a peripheral edge of test substance W is overlapped with X-ray which has passed through no test substance W on phosphor plate 7 as shown in Fig. 3, and X-ray intensity of the overlapped portion is enhanced. On the other hand, there is caused a phenomenon wherein X-ray intensity is lowered by an amount equivalent to the portion of refracted X-ray. Therefore, edge-enhancement function (which is called also as edge effect) wherein a difference of X-ray intensity is broadened on the boundary of the peripheral edge of test substance W, operates, and the radiograph having high visibility in which a peripheral edge portion is described sharply can be obtained.

When the X-ray source 2 is regarded as a dotted line source, X-ray intensity on the peripheral edge portion results in one shown with solid lines in Fig. 4. E shown in Fig. 4 represents a half band width of edge-enhancement, and it can be obtained by the following expression (2). The half band width E represents a distance between a peak and a trough of an edge.

[Numeral 1]

$$E = 2.3 \left(1 + \frac{R2}{R1}\right)^{\frac{1}{3}} \cdot \left\{R2 \cdot \delta \cdot (2r)^{\frac{1}{2}}\right\}^{\frac{2}{3}} \qquad \cdots (2)$$

In the aforesaid expression, $\delta$ represents a refraction difference at the point where refraction of X-ray is caused, and $\gamma$ represents a radius of an object (photographic object).

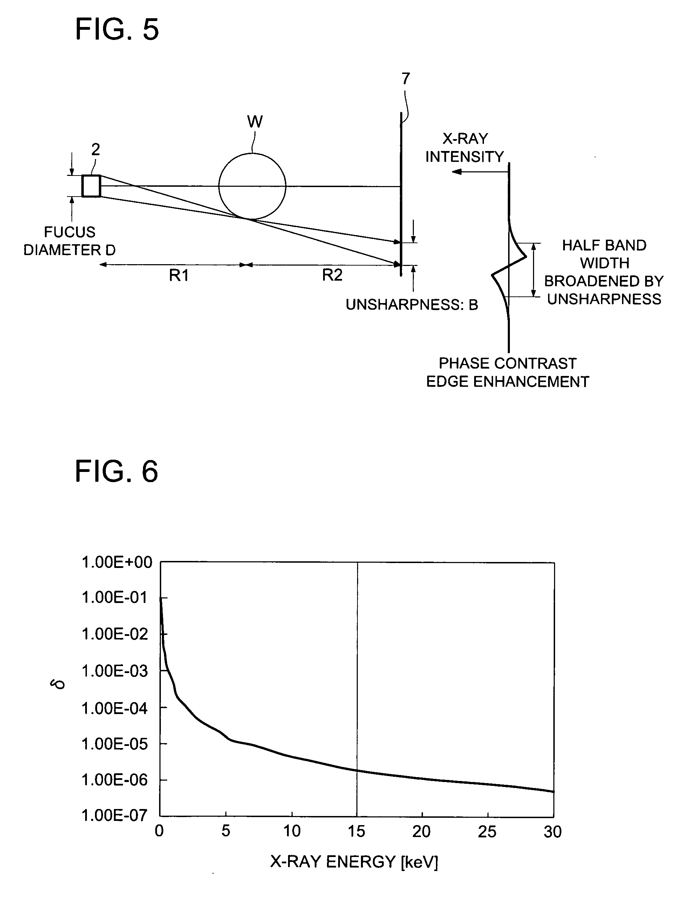

However, in the medical job site and the nondestructive inspection facilities, Coolidge X-ray tube (which is called also as hot-cathode X-ray tube) is widely used. This Coolidge X-ray tube cannot be regarded as an ideal dotted line source because focus size D grows greater to a certain extent as shown in Fig. 5. In this case, as shown in Fig. 5, ). the half band width E for edge-enhancement is broadened, and the intensity is lowered, resulting in a geometric blur. This geometric blur (geometric unsharpness) is called unsharpness.

X-ray intensity in the peripheral edge portion in the case of occurrence of unsharpness is one shown with dotted lines in Fig. 4. The half band width E for edge-enhancement portion in the case of occurrence of unsharpness is broadened to be wider than edge-enhancement width E in the case of assuming an ideal dotted line source, because of geometric blur. When FB represents an edge-enhancement half band width in the case of occurrence of unsharpness, EB can be obtained from the following expression (3).

[Numeral 2]

$$EB = 2.3 \left(1 + \frac{R2}{R1}\right)^{\frac{1}{3}} \cdot \left\{R2 \cdot \delta \cdot (2r)^{\frac{1}{2}}\right\}^{\frac{2}{3}} + D \cdot \frac{R2}{R1} \qquad \cdots (3)$$

In the aforesaid expression, definitions of $\delta$ and $\gamma$ are the same as those in expression (2).

Further, EB is edge-enhancement half band width E in the case of no unsharpness plus B showing a size of unsharpness, and it is shown as EB = E + B.

<Conditions of X-ray energy>

The reason why X-ray radiated from X-ray source 2 is made to be low X-ray energy in a range from 10keV to 20keV is to enhance this phase contrast effect.

Fig. 6 is a graph showing relationship between X-ray energy of X-ray to be irradiated and refraction difference $\delta$ of X-ray generated on a peripheral edge of test substance W. This $\delta$ is a value which has an influence on a size of a phase shift, and it shows that the greater this $\delta$ is, the greater the phase contrast effect is.

As shown in Fig. 6, the lower the X-ray energy is, the greater the $\delta$ is. In other words, it is understood that the lower the X-ray energy is, the greater the phase contrast effect is.

It is therefore preferable to irradiate X-ray having low energy for increasing phase contrast and for enhancing sharpness of a radiograph. In the case of low energy, it is necessary to take a long time for irradiation for obtaining sufficient contrast in a radiograph. When an object to be radiographed is a human body, an X-ray irradiation for a long time must be avoided in view of a problem of an exposure dose. However, in the present embodiment, an exposure dose is not a problem in the present embodiment, because an object to be radiographed is test substance W.

When low energy is used, X-ray is absorbed by an air layer, front plate 62a of image recording section 6 and a protective film of phosphor plate 7 during the period for X-ray to arrive at phosphor plate 7 from X-ray source 2, thereby, an amount of X-ray arriving at phosphor plate 7 is reduced. When an arrived X-ray amount is less than 45%, a noise on a radiograph grows greater, and it becomes difficult to distinguish a noise and a shade of asbestos.

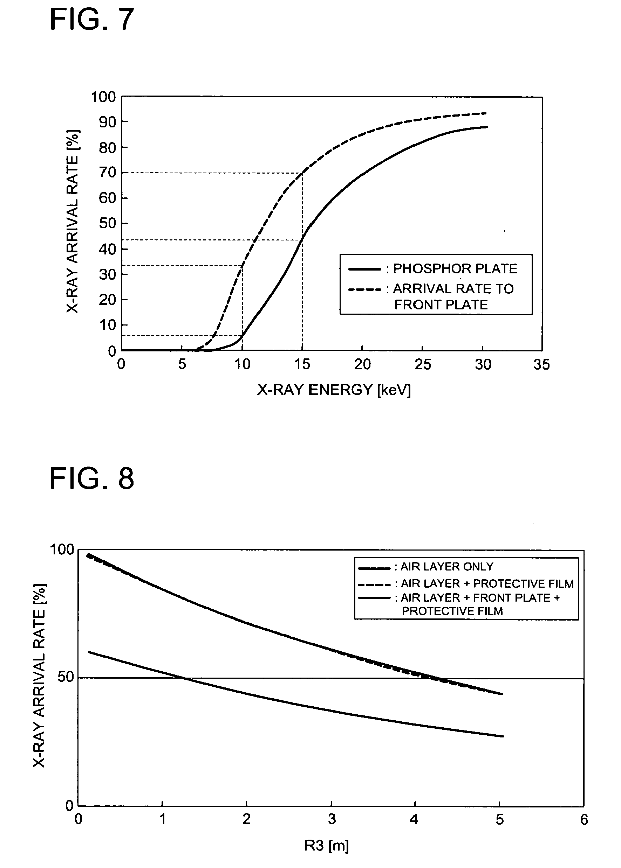

In Fig. 7, in the case that X-rays are directly emitted from the X-ray source 2 to the phosphor plate 7 without placing any test substance between them, the relationship between X-ray energy of the X-rays emitted from the X-ray source 2 and a X-ray arrival ratio of an amount of X-rays having arrived to the phosphor plate 7 to an amount of the X-rays emitted from the X-ray source 2 is shown with solid lines, and the relationship between X-ray energy and an X-ray arrival ratio to the front plate 62a is shown with dotted lines. As shown in Fig. 7, when X-ray energy is 10 keV, X-ray arrival ratio up to front plate 62a is as low as about 30% and X-ray arrival ratio up to phosphor plate 7 is about 6%, and a value of an amount of X-ray detected finally is small. Under this condition, since X-ray needs to be irradiated for a long time for obtaining image density that allows a radiograph to be observed, the X-ray energy is preferably 10keV or more.

On the other hand, when X-ray energy is 15 keV, X-ray arrival ratio in front plate 62a is about 70% and X-ray arrival ratio in phosphor plate 7 is about 45%. For example, under the conditions wherein magnifying power M is 20, focus size D is 10, and image recording section 6 equipped with front plate 62a having X-ray absorption factor of 5% is used, when obtaining image quality identical to that in the case of irradiating X-ray energy of 15keV, by irradiating X-ray energy of 10keV, radiographing time that is about 9.2 times that in the occasion of 15 keV is required.

With the consideration for the radiographing time, it may be preferable to make the X-ray energy larger. However, as explained in Fig. 6, the larger the X-ray energy is, the smaller the phase contrast effect is. Therefore, with the consideration for the phase contrast effect, the X-ray energy is preferably 20keV or less.

Therefore, from the viewpoint of the balance for an improvement of the phase contrast effect, the X-ray arrival ratio and the radiographing time, X-ray energy is preferably in a range of from 10keV to 20keV, more preferably in a range of from 10keV to 15keV.

Incidentally, in the present invention, the X-ray energy means "X-ray average energy", and the X-ray average energy can be obtained the value calculated by "total emitted X-ray energy / total emitted photons".

<Condition of radiographing arrangement, structure of image recording section>

For enhancing X-ray arrival ratio to phosphor plate 7, it is necessary to eliminate items absorbing X-ray as far as possible from a space between X-ray source 2 and phosphor plate 7.

In this case, an item which could be a factor to absorb X-ray in a space between X-ray source 2 and phosphor plate 7 including test substance W includes front plate 62a, a protective film of phosphor plate 7 and an air layer. The table 8 as the test substance holding member on which test substance W is placed can also be the factor. However, as described above, it is preferably to avoid X-ray absorption by the table 8 by providing the opening 9 in the table 8 as shown in Fig. 1(b).

As shown by the graph in Fig. 7, when irradiating X-ray energy of 15 keV, X-ray arrival ratio up to front plate 62a is about 70% and X-ray arrival ratio up to phosphor plate 7 is about 45%. This tells that about 30% of X-ray energy is absorbed by an air layer covering from X-ray source 2 to front plate 62a, and about 25% of X-ray energy is absorbed by front plate 62a of phosphor plate 7 and by a protective film of phosphor plate 7. Namely, an air layer, front plate 62a and a protective film of phosphor plate 7 are regarded as main factors to worsen X-ray arrival ratio.

Therefore, in the X-ray radiographing apparatus 1, cassette 61 wherein a thickness of front plate 62a representing a factor to absorb X-ray during radiographing is adjusted to be thin is used to reduce X-ray absorption factor by front plate 62a, or the front plate 62a is removed for radiographing, so that X-ray arrival ratio up to phosphor plate 7 may be 45% or more, preferably 50% or more. On the other hand, radiographing distance R3 is established to be a short distance to reduce X-ray absorption factor concerning an air layer.

When the human body is an object to be radiographed, resistance characteristics and strength against a load are required for the front plate 62a, because a portion of the human body is made to come in contact with image detector 6 directly in many cases, and the front plate is usually constructed to have a certain thickness. However, when phase contrast radiographing is conducted with test substance W representing an object to be radiographed, it is not considered that a patient comes in contact directly with image detector 6, and it is sufficient that a light shielding function is provided. Therefore, even if the strength is declined by the reduced thickness of the front plate 62a, there is no problem for radiographing. This also applies to the occasion where the front plate 62a is removed.

On the other hand, radiographing distance R3 (distance between the X-ray source 2 and the phosphor plate 7) needs to be established, in consideration of the balance for an irradiation (exposure field) and an X-ray arrival ratio.

Fig. 8 shows relationship between the radiographing distance R3 and the X-ray arrival ratio in phosphor plate 7.

In Fig. 8, there are shown X-ray arrival ratio in the case where an air layer only is allowed to lie between X-ray source 2 and phosphor plate 7, X-ray arrival ratio in the case where an air layer and a protective film of the phosphor plate 7 only are allowed to lie between X-ray source 2 and phosphor plate 7, and X-ray arrival ratio in the case where an air layer, front plate 62a and a protective film of the phosphor plate 7 are allowed to lie between X-ray source 2 and phosphor plate 7. Incidentally, characteristics in the case of an air layer only and those in the case of an air layer and a protective film only are substantially the same, and lines showing them are indicated to be superimposed each other.

In the mean time, in the case of measurement for the radiographing distance R3 and for X-ray arrival ratio, front plate 62a made of carbon is used, and a thickness of the front plate 62a is adjusted so that X-ray absorption factor of the carbon may be the same as the X-ray absorption factor in the case where a thickness of aluminum is 0.5 mm. A PET film having a thickness of 50 $\mu$m is used for a protective film of phosphor plate 7. Further, magnifying power M in the case of phase contrast radiographing is 20, and energy of X-ray used for irradiation is 15 keV.

As is shown in Fig. 8, when an air layer and a protective film intervene, X-ray arrival ratio up to phosphor plate 7 is 45% or more under the condition of R3 < 4. This is a result identical to that in the case of an air layer only. It is therefore understood that a protective film of phosphor plate 7 is not a factor that worsens X-ray transport to phosphor plate 7.

When front plate 62a lies in addition to the air layer and the protective film, X-ray arrival ratio is 45% or more under the condition of R3 < 1.2.

On the other hand, the smaller R3 is, the higher the X-ray arrival ratio is, but when R3 is excessively small, distance R1 from X-ray source 2 to test substance needs to be small inevitably. If R3 is less than 0.5 m, an irradiation is narrowed extremely, and the irradiation cannot include a portion to be observed accordingly, resulting in occurrence of a deficit in a radiograph, which has been known.

Therefore, when radiographing after removing front plate 62a, distance R3 is established to be within a range of 0.5 < R3 < 4. On the other hand, when radiographing after mounting front plate 62a, distance R3 is established to be within a range of 0.5 < R3 < 1.2. By setting R3 to be within that range, it is possible to obtain X-ray arrival ratio of 45% or more and to secure the irradiation necessary for observation.

Meanwhile, when front plate 62a is mounted, it is sometimes possible to attain X-ray arrival ratio of 45% or more, by adjusting a material or a thickness of front plate 62a, even in the case of $1.2 \leq R3$. Namely, X-ray absorption factor of front plate 62a can be reduced by changing the material of front plate 62a to a material having lower X-ray absorption factor, or by reducing the thickness of front plate 62a, by an amount equivalent to the percentage by which the X-ray absorption factor in phosphor plate 7 is lowered from 45% by the condition of $1.2 \leq R3$.

A thickness of front plate 62a can be determined based on characteristics shown in Fig. 8, from relationship with radiographing distance R3. For example, in the case of R3 = 3, X-ray arrival ratio to phosphor plate 7 in the case of interposing of an air layer, front plate 62a and a protective film, is 35%. Therefore, for making X-ray arrival ratio to phosphor plate 7 to be 45% or more, a thickness or a material of front plate 62a may be adjusted so that X-ray absorption factor by front plate 62a may be reduced by 15%.

<Magnifying power, condition of focus size>

For improving visibility of a microscopic object to be radiographed having a diameter of 50 ($\mu$m) or less such as asbestos, it is necessary to enhance magnifying power M. For enhancing magnifying power M, distance R2 (distance between a test substance W and the image recording section 6) needs to be increased, which is derived by expression (1), but, an increase of distance R2 causes enlargement of a half band width EB of unsharpness. From now on, a diameter of an object to be radiographed will be represented by s ($\mu$m). Diameter s represents a diameter of a circumcircle on the occasion where an object is not a heteromorphic body but is substantially spherical or substantially a cube, and it represents a diameter of a section in the direction perpendicular to the extended direction (longitudinal direction) of a heteromorphic body on the occasion where an object is a heteromorphic body such as a thread-shaped long and slender one.

Further, as is understood from expression (3), an extent of unsharpness B depends greatly on focus size D. When observing, on a radiograph, a microscopic object of $0.05 \leq s \leq 50$ ($\mu$m) such as asbestos, if focus size D is made to be greater, an amount of X-ray transport is increased and visibility of a radiograph is improved. However, an extent of unsharpness is enhanced that much, resulting in an image on which an edge-enhancement function is not obtained.

In this connection, for radiographing a microscopic object of $0.05 \leq s \leq 50$ ($\mu$m), focus size D of X-ray source 2 is made to be $1 \leq D \leq 30$ ($\mu$m) and 1magnifying power M is made to be $10 \leq M \leq 40$, which makes it possible to obtain excellent visibility on a radiograph.

Each of the following Table 1 and Table 2 shows results of vision evaluation conducted for radiographs acquired by experimental phase contrast radiographing.

In the experiments, phase contrast radiographing was conducted by using a photographic object representing a pseudo phantom wherein diameter s of a glass wool fiber was varied stepwise up to 0.05 - 50 ($\mu$m), and radiographs thus obtained were outputted on films to be subjected to vision evaluation.

The experiments were conducted under the following radiographing conditions.

X-ray source: The X-ray sources manufactured for trial by Konica Minolta Holdings, Inc. wherein focus sizes D are 10 (μm) and 30 (μm) were used. With respect to the X-ray radiographing apparatus, the trial product made by the same company was used.

Image recording section: Regius plate RP-5PM representing a phosphor plate made by the same company and Regius cassette RC-110M (which is a cassette having the same structure disclosed in the aforesaid Japanese Patent Publication Open to Public Inspection No. 2002-156717) were used.

Reading of radiograph from the image recording section: Reading was conducted by Regius model 190 made by the same company under the condition of reading pixel pitch of 43.75 (μm).

Image processing: After the reading, image processing to adjust the contrast of a radiograph with Regius console (made by the same company) was conducted. Parameter G value relating to the adjustment of contrast was made to be 20 (G value for ordinary radiographing of human body is 3 - 5). The G value is one to be adjusted on the basis of "the greater the G value is, the higher the contrast is".

Output of images read to film: Output was conducted by Drypro model 793 made by the same company under the condition of writing pixel pitch of 25 (μm). In this case, the output was conducted by making each pixel of images read and each pixel of outputted images to be on a one to one correspondence, without conducting interpolation processing.

The evaluation criteria are as follows.

A: Each bristle of the fiber can be recognized clearly.
B: Existence of the fiber can be confirmed.
C: Existence of the fiber cannot be confirmed.

Seven estimators observed images of glass wool fiber on the film to evaluate them.

Table 1

| No. | Magnifying power M | R1[m] | R2[m] | Focus size D [μm] | Unsharpness B [μm] | Edge-enhancement width EB [μm] | Decision |
|-----|--------------------|-------|-------|-------------------|--------------------|--------------------------------|----------|
| 11 | 1 | 2 | 0 | 10 | 0 | 0 | C |
| 12 | 1.75 | 2 | 1.5 | 10 | 7.5 | 28.6 | C |
| 13 | 5 | 0.7 | 2.8 | 10 | 40 | 68.9 | C |
| 14 | 10 | 0.35 | 3.15 | 10 | 90 | 129.5 | B |
| 15 | 15 | 0.23 | 3.27 | 10 | 140 | 198.8 | B |
| 16 | 20 | 0.175 | 3.33 | 10 | 190 | 241.7 | A |
| 17 | 35 | 0.1 | 3.4 | 10 | 340 | 403.2 | A |
| 18 | 40 | 0.0875 | 3.413 | 10 | 390 | 456.2 | B |
| 19 | 50 | 0.07 | 3.43 | 10 | 490 | 561.5 | C |

Table 2

| No. | Magnifying power M | R1[m] | R2 [m] | Focus size D [μm] | Unsharpness B [μm] | Edge-enhancement width EB [μm] | Decision |
|-----|--------------------|-------|--------|-------------------|--------------------|--------------------------------|----------|
| 21 | 1 | 2 | 0 | 30 | 0 | 0 | C |
| 22 | 1.75 | 2 | 1.5 | 30 | 22.5 | 43.6 | C |
| 23 | 5 | 0.7 | 2.8 | 30 | 120 | 148.9 | C |
| 24 | 10 | 0.35 | 3.15 | 30 | 270 | 309.5 | B |
| 25 | 15 | 0.23 | 3.27 | 30 | 420 | 478.8 | A |
| 26 | 20 | 0.175 | 3.33 | 30 | 570 | 621.7 | A |

(continued)

| No. | Magnifying power M | R1[m] | R2 [m] | Focus size D [μm] | Unsharpness B [μm] | Edge-enhancement width EB [μm] | Decision |
|---|---|---|---|---|---|---|---|
| 27 | 35 | 0.1 | 3.4 | 30 | 1020 | 1083.2 | B |
| 28 | 40 | 0.0875 | 3.413 | 30 | 1170 | 1236.2 | B |
| 29 | 50 | 0.07 | 3.43 | 30 | 1470 | 1541.5 | C |

Incidentally, No. 11 of each of Table 1 and Table 2 shows the results of ordinary radiographing under the condition of magnifying power M = 1 conducted for comparing with phase contrast radiographing.

Further, unsharpness B and edge-enhancement width EB in each of Table 1 and Table 2 are calculated from expression (2) and expression (3).

Although diameter s of glass wool fiber was changed within a range of 0.05 - 50 (μm), all diameters s showed the same image quality, and the same results of evaluation were obtained. Therefore, evaluation items in Table 1 and Table 2 show the evaluation of image quality for the glass wool fiber of all diameters s.

From Table 1 and Table 2, it is apparent that image quality with high visibility for glass wool fiber (evaluation is B or A) is obtained when focus size D is within a range of $1 \leq D \leq 30$ and magnifying power M is within a range of $10 \leq M \leq 40$. Further, the magnifying power M is preferably within a range of $15 \leq M \leq 4$, more preferably within a range of $18 \leq M \leq 28$.

<Radiographing method>

Next, a radiographing method of the aforesaid X-ray radiographing apparatus 1 will be explained.

First, an occasion for radiographing after removing front plate 62a will be explained.

A radiographer fixes the position for radiographing test substance W. Then, he or she sets image recording section 6 housing therein phosphor plate 7 to be used for radiographing on holding section 5. After the image recording section 6 is set on the holding section 5, the radiographing room is changed to the dark room, and front plate 62a is removed.

After completion of the aforesaid preparation for radiographing, an operation to instruct the start of radioraphing is carried out in the X-ray radiographing apparatus 1.

In the X-ray radiographing apparatus 1, distance R1 and distance R2 each causing magnifying power M to be $10 \leq M \leq 40$ are calculated within a range of 0.5 < R3 < 4 for radiographing distance R3, and radiographing section 3 and holding section 5 are moved respectively to height positions for the distance R1 and distance R2. Meanwhile, this movement may also be carried out based on manual operations of the radiographer. Then, X-rat energy of 15 keV or less is irradiated from X-ray source 2 through focus size D of $1 \leq D \leq 30$. With respect to a period of time for radiographing, it is possible to employ the radiographing time that offers the best density from relationship between X-ray energy and density of a radiograph obtained in advance by irradiation of that X-ray energy.

During the period of radiographing, the radiographing room is kept to be a dark room constantly, to avoid light invasion to phosphor plate 7. After completion of radiographing, the radiographer takes image recording section 6 out of the holding section, after mounting front plate 62a on phosphor plate 7. After mounting the front plate 62a, operations can be done by changing the radiographing room to a daylight room. Then, the image recording section 6 which has been subjected to radiographing is loaded in a reading apparatus so that processing of reading of a radiograph from phosphor plate 7 in the image recording section 6.

Next, an occasion for radiographing while the front plate 62a is mounted will be explained.

In this case, a material and a thickness of the front plate 62a are determined in advance from the radiographing distance R3 and from relationship with the radiographing distance R3, so that X-ray arrival ratio on phosphor plate 7 may be 45% or.more. The radiographing distance R3 is determined to be within a range of 1 < R3 < 4, and there is prepared cassette 61 wherein a material and a thickness of front plate 62a are adjusted so that X-ray arrival ratio on phosphor plate 7 in the case of the determined radiographing distance R3 may be 45% or more.

The radiographer puts phosphor plate 7 in the aforesaid cassette 61, and mounts the cassette on the holding section 5. In this case, it is possible to keep the radiographing room to be a daylight room constantly, because the phosphor plate 7 is shielded by front plate 62a, which is different from the occasion to remove the front plate 62a.

In the X-ray radiographing apparatus 1, distance R1 and distance R2 each causing magnifying power M to be $10 \leq M \leq 40$ are calculated under the condition of the radiographing distance R3 determined in advance from the relationship with front plate 62a of cassette 61, and radiographing section 3 and holding section 5 are moved respectively to height positions for the distance R1 and distance R2 respectively. Meanwhile, this movement may also be carried out based on manual operations of the radiographer. Then, X-ray energy of 15 keV is irradiated from X-ray source 2 through focus size D of $1 \leq D \leq 30$. Operations of the radiographer after radiographing are the same as those in the occasion where the front plate 62a was removed.

In the meantime, it is also possible to conduct radiographing in the same way as in the foregoing, even in the case of employing FPD as an X-ray detector. In this case, again, it is necessary to make the radiographing room to be a dark room, while the casing portion is removed.

**EXAMPLES**

In the X-ray radiographing apparatus, phase contrast radiographing was conducted under the following experimental conditions, and the radiographs thus obtained were outputted to films to be outputted images which were subjected to vision evaluation.

<Experimental condition>

Test substance: Glass wool wherein a diameter of a bristle was varied stepwise within a range of 0.05 - 10 ($\mu$m) was pasted on an acrylic plate having a thickness of 5 cm, and this was used as a pseudo phantom of asbestos.

X-ray radiographing apparatus, X-ray source: X-ray radiographing apparatus manufactured for trial by Konica Minolta Holdings, Inc. was used. As an X-ray source, a W (tungsten) micro-focus X-ray source that emits X-ray with focus size D = 10 ($\mu$m) which was manufactured for trial by the same company was used.

Image recording section: As a phosphor plate, Regius plate RP-5PM made by the same company was used. As a cassette for housing therein a phosphor plate, a production prototype of the same company was used.

Reading apparatus: Regius model 190 made by the same company was used.

Image processing: After the reading, image processing to adjust contrast of a radiograph with Regius console (made by the same company) was conducted. Parameter G value relating to the contrast was made to be 20 (G value in the case of radiographing an ordinary human body is 3 - 5). The G value is one to be adjusted on the basis of the greater the G value is, the higher the contrast is.

Output apparatus: DRYPRO model 793 made by the same company was used.

<Radiographing condition 1>

Image quality in the case of changing radiographing distance R3 under the following radiographing conditions was evaluated.

X-ray tube voltage: 30 keV (Average X-ray energy 14.89keV)
X-ray tube current: 1 mA
Magnifying power: M = 20
Radiographing arrangement: R3 was varied within a range of 0.5 - 5, and R1 was varied within a range of 0.025 - 0.25 so that M may be 20 in accordance with R3.

Image recording section: A material of the front plate of the cassette was made to be carbon, and the thickness of the front plate was adjusted to be of a thickness that makes X-ray absorption factor to be 5%.

<Radiographing condition 2>

In the radiographing condition 1, image quality was evaluated by changing magnifying power M to M = 40. However, radiographing distance R3 was varied within a range of 1 - 4, and R1 and R2 were changed to attain M = 40 in accordance with R3.

<Radiographing condition 3>

In the radiographing condition 1, image quality was evaluated by changing magnifying power M to M = 10. However, radiographing distance R3 was varied within a range of 0.5 - 4, and R1 and R2 were changed to attain M = 10 in accordance with R3.

<Radiographing condition 4>

Under the following radiographing conditions, there was conducted evaluation of image quality in the case of changing a material and X-ray absorption factor of the front plate.

**EP 1 879 021 A2**

X-ray tube voltage: 30 keV (Average X-ray energy 14.89 keV)
X-ray tube current: 1 mA

Magnifying factor, radiographing arrangement: M = 20 was fixed, and R1 = 0.1, R2 = 1.9 and R3 = 2.0 were fixed.

Image recording section: With respect to front plates of the cassette, those made of carbon and those made of aluminum were prepared. Further, the front plates were adjusted in terms of a thickness and they were changed in terms of X-ray absorption factor within a range of 5 - 70%, to be used for radiographing. As a protective film of the phosphor plate, PET film having a thickness of 20 $\mu$m (X-ray absorption factor in 15 keV is 0.3%) was used.

<Measurement of X-ray absorption factor>

An X-ray measuring instrument wad installed at a position immediately before the installation location of a phosphor plate of the image recording section, and X-ray energy was measured by the X-ray measuring instrument. The X-ray absorption factor covering from the X-ray source to the phosphor plate was calculated from the measured X-ray energy (X-ray absorption factor is the quotient obtained by dividing the measured X-ray energy (keV) by 15 keV). Namely, the X-ray arrival ratio to phosphor plate is the remainder obtained by subtracting the X-ray absorption factor from 100%. Therefore, when the calculated X-ray absorption factor is less than 50%, the X-ray arrival ratio in the phosphor plate is 45% or more.

<Evaluation criteria>

Evaluation criteria for the radiograph outputted to be formed on the film are as follows.

AA: Each bristle of the fiber can be recognized clearly.
A: A group of several bristles of the fiber can be recognized.
B: Existence of a lump of bristles of the fiber can be recognized.
C: A fiber is not visible.

Seven image estimators observed images on the film, and evaluated images of glass wool fiber representing an object to be radiographed in accordance with the aforesaid evaluation criteria.

<Results of the evaluation>

Table 3 shows the results of the evaluation of radiographs obtained through the Radiographing condition 1. Table 4 shows the results of the evaluation of radiographs obtained through the Radiographing condition 2. Table 5 shows the results of the evaluation of radiographs obtained through the Radiographing condition 3. Table 6 shows the results of the evaluation of radiographs obtained through the Radiographing condition 4.

**12**

Table 3

| No. | Distance for radiographing R3 [m] | Magnifying power M | R1[m] | R2[m] | Focus diameter D[$\mu$m] | Front plate | | X-ray absorption factor [%] | Exposure field evaluation | Evaluation |
|---|---|---|---|---|---|---|---|---|---|---|
| | | | | | | Material | X-ray absorption factor | | | |
| 31 | 0.5 | 20 | 0.025 | 0.475 | 10 | Carbon | 5% | 12.9948 | Partial loss | A |
| 32 | 1 | 20 | 0.05 | 0.95 | 10 | Carbon | 5% | 20.05107 | Excellent | A |
| 33 | 2 | 20 | 0.1 | 1.9 | 10 | Carbon | 5% | 32.51556 | Excellent | AA |
| 34 | 3 | 20 | 0.15 | 2.85 | 10 | Carbon | 5% | 43.0384 | Excellent | AA |
| 35 | 4 | 20 | 0.2 | 3.8 | 10 | Carbon | 5% | 51.91319 | Excellent | B |
| 36 | 5 | 20 | 0.25 | 4.75 | 10 | Carbon | 5% | 59.41462 | Excellent | C |

Table 4

| No. | Distance for radiographing R3 [m] | Magnifying power M | R1[m] | R2[m] | Focus diameter D [μm] | Front plate | | X-ray absorption factor [%] | Exposure field evaluation | Evaluation |
|---|---|---|---|---|---|---|---|---|---|---|
| | | | | | | Material | X-ray absorption factor | | | |
| 52 | 1 | 40 | 0.025 | 0.95 | 10 | Carbon | 5% | 20.05107 | Excellent | B |
| 53 | 2 | 40 | 0.05 | 1.95 | 10 | Carbon | 5% | 32.51556 | Excellent | A |
| 54 | 3 | 40 | 0.1 | 2.9 | 10 | Carbon | 5% | 43.0384 | Excellent | A |
| 55 | 4 | 40 | 0.15 | 3.85 | 10 | Carbon | 5% | 51.91319 | Excellent | B |
| 56 | 5 | 40 | 0.2 | 4.8 | 10 | Carbon | 5% | 59.41462 | Excellent | C |

Table 5

| No. | Distance for radiographing R3 [m] | Magnifying power M | R1[m] | R2[m] | Focus diameter D [μm] | Front plate | | X-ray absorption factor [%] | Exposure field evaluation | Evaluation |
|---|---|---|---|---|---|---|---|---|---|---|
| | | | | | | Material | X-ray absorption factor | | | |
| 61 | 0.5 | 10 | 0.05 | 0.45 | 10 | Carbon | 5% | 12.9948 | Partial loss | B |
| 62 | 1 | 10 | 0.1 | 0.9 | 10 | Carbon | 5% | 20.05107 | Excellent | B |
| 63 | 2 | 10 | 0.2 | 1.8 | 10 | Carbon | 5% | 32.51556 | Excellent | A |
| 64 | 3 | 10 | 0.3 | 2.7 | 10 | Carbon | 5% | 43.0384 | Excellent | A |
| 65 | 4 | 10 | 0.4 | 3.6 | 10 | Carbon | 5% | 51.91319 | Excellent | B |
| 66 | 5 | 10 | 0.5 | 4.5 | 10 | Carbon | 5% | 59.41462 | Excellent | C |

Table 6

| No. | Distance for radiographing R3 [m] | Magnifying power M | R1[m] | R2[m] | Focus diameter D[$\mu$m] | Front plate | | X-ray absorption factor [%] | Evaluation |
|---|---|---|---|---|---|---|---|---|---|
| | | | | | | Material | X-ray absorption factor | | |
| 41 | 2 | 20 | 0.1 | 1.9 | 10 | Aluminum | 5% | 32.51556 | AA |
| 42 | 2 | 20 | 0.1 | 1.9 | 10 | Aluminum | 10% | 36.06738 | AA |
| 43 | 2 | 20 | 0.1 | 1.9 | 10 | Aluminum | 30% | 50.27463 | A |
| 44 | 2 | 20 | 0.1 | 1.9 | 10 | Aluminum | 50% | 64.48188 | C |
| 45 | 2 | 20 | 0.1 | 1.9 | 10 | Aluminum | 70% | 78.68913 | C |
| 46 | 2 | 20 | 0.1 | 1.9 | 10 | Carbon | 10% | 36.06738 | AA |
| 47 | 2 | 20 | 0.1 | 1.9 | 10 | Carbon | 50% | 64.48188 | C |

Incidentally, there was no difference of visibility even if diameter s was changed in a range of 0.05 - 10 ($\mu$m), in the case of glass wool fiber. Evaluation results in each of Table 3 and Table 4 indicate results of the evaluation for glass wool fibers covering all ranges of different diameters s.

As is apparent from Table 3, a range of 0.5 (m) - 4 (m) for the radiographing distance R3 creates image quality wherein a glass wool fiber is visible. In the aforesaid range, a range of 2 (m) - 3 (m) for R3 shows high visibility. The reason for this is an improvement of sharpness on the peripheral edge portion caused by a phase contrast effect, and it is considered that the phase contrast effect was enhanced by changing X-ray energy to the low energy which is as low as 15 keV. Although the shorter the radiographing distance R3 is, the smaller the X-ray absorption factor is, if the distance R1 is less than 0.05 m, the irradiation is narrowed to cause partial loss, though image quality is good. Further, from the results of No. 36, it is understood that if the distance R3 exceeds 4 m, the X-ray absorption factor turns out to be about 60%, resulting in image quality in which the glass wool fiber is not visible.

Further, from Table 4 and Table 5, it is apparent that excellent image quality can be obtained by making magnifying power M to be within a range of $10 \leq M \leq 40$.

Further, in Table 6, it is understood, from the results of 41 - 43, that X-ray absorption factor can be adjusted to be about 50% or less and excellent image quality can be obtained, if a thickness of the front plate made of aluminum is adjusted so that X-ray absorption factor of the front plate may be 5% - 30%. In contrast to this, in No. 44 and No. 45, X-ray absorption factor of the front plate exceeds 60% and a glass wool fiber is not visible. In this connection, from the results of No. 47, it can be said that the same thing applies to the occasion where the material is changed to carbon.

On the other hand, from the results of No. 3 of Table 3 and the results of No. 6 of Table 6, it is understood that images having high sharpness can be obtained even when a material of the front plate is made to be carbon, if X-ray absorption factor of the front plate is made to be 5 - 10 (%) .

As stated above, in the present embodiment, since the asbestos having diameter s of $0.05 \leq s \leq 50$ is an object to be radiogphed, X-ray having energy of 15keV or lower is irradiated at focus size D of $1 \leq D \leq 30$, then, phase contrast radiographing at magnifying power M of $10 \leq M \leq 40$ is conducted, and the structure of front plate 62a of cassette 61 and/or radiographing distance R3 is adjusted so that X-ray arrival ratio to phosphor plate 7 may be 45% or more. Namely, R3 is adjusted to be within a range of $0.5 < R3 < 4$ so that X-ray arrival ratio to phosphor plate 7 may be 45% or more, and radiographing is carried out by using cassette 61 from which the front 62a is removed. Or, a material and a thickness of the front plate 62a are adjusted to reduce X-ray absorption factor of the front plate 62a.

Phase contrast radiographing is conducted under the conditions of the aforesaid focus size D, X-ray energy, magnifying power M and radiographing distance R3, and further, the structure of the front plate 62a is adjusted as stated above. Thereby, an phase contrast effect obtained by the phase contrast radiographing can be enhanced, and an increase of X-ray arrival ratio to phosphor plate 7 can be attained as far as possible. Therefore, even a microscopic object to be radiographed such as asbestos can be recognized clearly on a radiograph, and image quality having sufficient contrast can be obtained.

Further, by making the distance for radiographing to satisfy $2 < R3 < 3$, it is possible to increase the X-ray arrival ratio to phosphor plate 7 and to attain image quality wherein visibility of asbestos in a radiograph is high.

**Claims**

1. An X-ray radiographing apparatus, comprising:

    (1) a radiographing section having an X-ray source provided with a focus size D ($\mu$m) of from 1 to 30 and for emitting X-rays having an X-ray energy (keV) of from 10 to 20;
    (2) a test substance holding section for holding a test substance;
    (3) an image recording section for accommodating a X-ray detector;

    wherein the X-ray source, the test substance holding section and the image recording section are arranged and structured to conduct a phase contrast radiographing such that when R1 represents a distance (m) from a focal point of the X-ray source to the test substance held by the test substance holding section and R3 represents a distance (m) from the focal point of the X-ray source to the X-ray detector, a magnifying power M represented by the formula of (M = R3/R1) is from 10 to 40 and an X-ray arrival ratio of an amount of X-rays having arrived to the X-ray detector to an amount of the emitted X-rays is 45 % or more.

2. The X-ray radiographing apparatus of claim 1, wherein the X-ray arrival ratio is 50 % or more.

3. The X-ray radiographing apparatus of claim 1, wherein the X-ray energy (keV) is from 10 to 15.

4. The X-ray radiographing apparatus of claim 1, wherein the image recording section is arranged to satisfy the following formula.

$$0.5 < R3 < 4 \ (m)$$

5. The X-ray radiographing apparatus of claim 4, wherein the image recording section is arranged to satisfy the following formula.

$$2 < R3 < 3 \ (m)$$

6. The X-ray radiographing apparatus of claim 1, wherein the magnifying power M is from 15 to 35.

7. The X-ray radiographing apparatus of claim 6, wherein the magnifying power M is from 18 to 28.

8. The X-ray radiographing apparatus of claim 1, wherein the test substance holding section and the image recording section are located below the radiographing section so as to make the distances R1 and R3 adjustable respectively.

9. The X-ray radiographing apparatus of claim 1, wherein the image recording section includes a casing, the casing has a front plate to cover a irradiation surface of the X-ray detector, and the front plate is structured to be removable at the time of the phase contrast radiographing.

10. The X-ray radiographing apparatus of claim 1, wherein the test substance holding section has an opening not to shield an X-ray irradiation region of the test substance.

11. The X-ray radiographing apparatus of claim 1, wherein the X-ray detector is a phosphor plate.

12. The X-ray radiographing apparatus of claim 1, wherein the X-ray detector is a semiconductor element plate.

13. The X-ray radiographing apparatus of claim 1, wherein the size ($\mu$m) of the test substance is 0.05 to 50.

FIG. 1 (a)

FIG. 1 (b)

# FIG. 2 (a)

61

63a
63b
63c
7
63c
63

62b
62a
62

# FIG. 2 (b)

63c  63b  62a  7  63c
62b
62c  63a  62c

FIG. 3

FIG. 4

# FIG. 5

W

7

2

X-RAY
INTENSITY

FUCUS
DIAMETER D

R1

R2

UNSHARPNESS: B

HALF BAND
WIDTH
BROADENED BY
UNSHARPNESS

PHASE CONTRAST
EDGE ENHANCEMENT

# FIG. 6

1.00E+00
1.00E-01
1.00E-02
1.00E-03
1.00E-04
1.00E-05
1.00E-06
1.00E-07

$\delta$

0      5      10      15      20      25      30

X-RAY ENERGY [keV]

## FIG. 7

## FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006194073 A
- JP 10268450 A
- WO 11502620 A
- JP 2002156717 A
- JP 2005106783 A